# EUROPEAN PATENT APPLICATION

(11) **EP 2 093 216 A1**
(43) Date of publication of application: **26.08.2009**
(21) Application number: 08151892.0
(22) Date of filing: 25.02.2008
(51) Int. Cl.: C07D 213/82, C07D 231/14

(54) **Process for preparing substituted biphenylanilides**

(71) Applicant: Bayer CropScience AG, 40789 Monheim (DE)
(72) Inventor: The designation of the inventor has not yet been filed

(57) **Abstract**

The present invention relates to a process for preparing substituted biphenylanilides of the formula 1 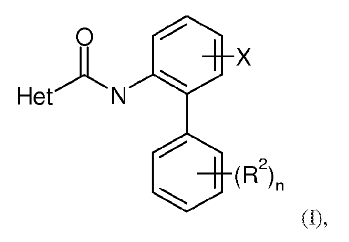 which comprises reacting a compound of formula II 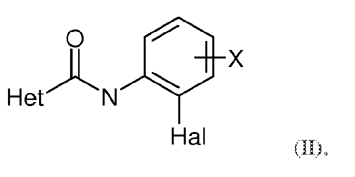 in the presence of a base and of a palladium catalyst selected from the group of: a) palladium-triarylphosphine or -trialkylphosphine complex with palladium in the zero oxidation state, b) salt of palladium in the presence of triarylphospine or trialkylphosphine as a complex ligand or c) metallic palladium, optionally applied to support, in the presence of triarylphosphine or trialkylphosphine, in a solvent, with a diphenylborinic acid according to formula (III)

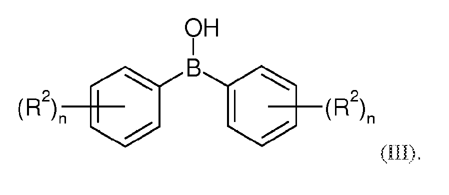

## Description

The present invention relates to a process for preparing substituted biphenylanilides of the formula I 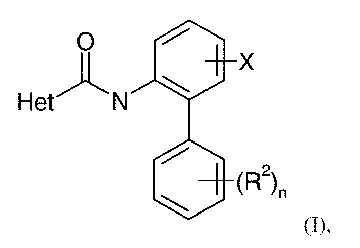 in which the substituents are defined as follows:
- Het: is a heterocyclyl-residue selected from 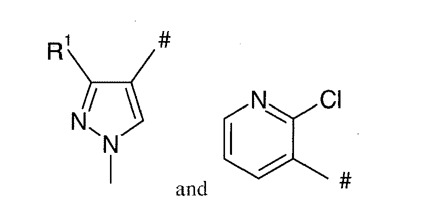 wherein "#" indicates the substituted position of the residue;
- X: is hydrogen fluorine or chlorine;
- R¹: is C₁-C₆-hatoalkyl.:
- R²: is cyano, nitro, halogen, C₁-C₆-alkyl, C₁-C₆-alkenyl, C₁-C₆-alkynyl, C₁-C₆-alkoxy, C₁-C₆-haloalkyl, (C₁-C₆-alkyl)carbonyl or phenyl;
- R³: is C₁-C₄-alky C₁-C₄-alkenyl or C₁-C₄-alkynyl;
- n: is 1, 2 or 3, where in case that n is 2 or 3, the R² radicals may also be different,
which comprises reacting a compound of formula II 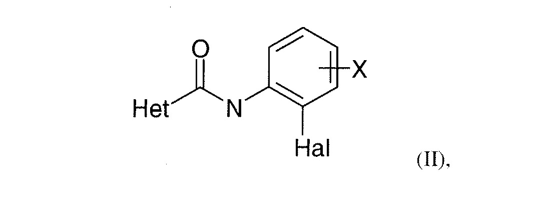 in which Hal is halogen and X and Het are as defined above, in the presence of a base and of a palladium catalyst selected from the group of: a) palladium-triarylphosphine or -trialkylphosphine complex with palladium in the zero oxidation state, b) salt of palladium in the presence of triarylphospine or trialkylphosphine as a complex ligand or c) metallic palladium, optionally applied to support, in the presence of triarylphosphine or trialkylphosphine, in a solvent, with a diphenylborinic acid according to formula (III) 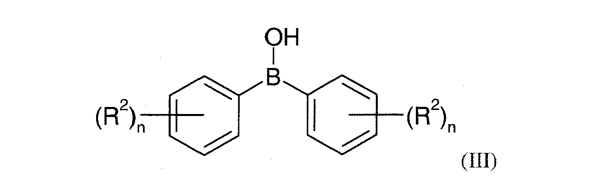 in which R² and n are as defined above, where the triarylphosphines or trialkylphosphines used may be substituted.

Tetrahedron Lett. 32, page 2277 (1991) states that the coupling reaction between phenylboronic acid and chlorobenzene with use of the [1,4-bis(diphenylphosphine)- butane]palladium(ll) dichloride catalyst proceeds with a yield of only 28%.

EP-A 0 888 261 discloses a process for preparing nitrobiphenyls by reacting chloronitrobenzenes with a phenylboronic acid in the presence of a palladium catalyst and of a base. In this process, a very high catalyst concentration is necessary.

WO 2006/092429 and WO 2007/138089 each pertain to a process for preparing substituted biphenyls by coupling substituted diphenylborinic acids with dihaloarylcompounds in presence of a palladium catalyst.

It was therefore an object of the present invention to provide an economically viable process which can be implemented on the industrial scale for regioselectively preparing substituted biphenylanilides, which works with a reduced palladium catalyst concentration.

Accordingly, the process defined at the outset has been found.

The diphenylborinic acid (III) is obtained by reaction of optionally substituted phenylmagnesium chloride V with trialkyl borate, preferably trimethyl borate, in tetrahydrofuran as a solvent according to WO 2007/138089 as described by scheme 1. 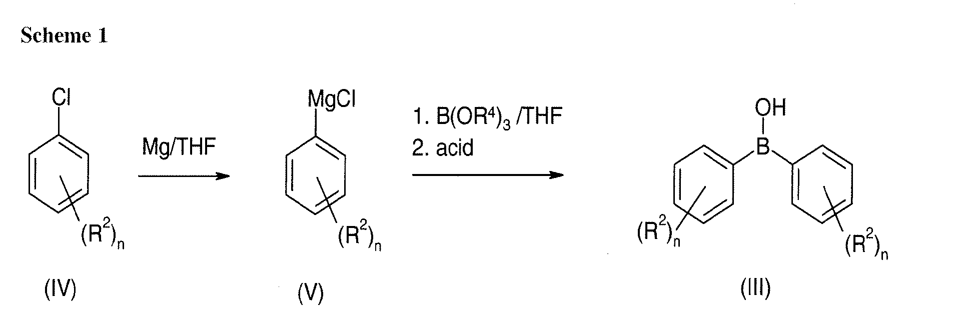
R⁴ is C₁-C₄-alkyl, preferably methyl.

Essential for a high yield of diphenylborinic acid (**III**) is the use of only 0.7 eq. of trialkyl borate based on the substituted chlorobenzene (IV) used. Use of 1.1 eq. of trialkyl borate gives rise to phenylboronic acid as described in EP-A 0 888 261.

The reaction temperature in this process stage is from 10 to 30°C, preferably from 15 to 25°C.

The substituted biphenyls prepared by the present process have the following preferred substituents, in each case both individually and in combination.
- Het: is a heterocyclyl -residue selected from 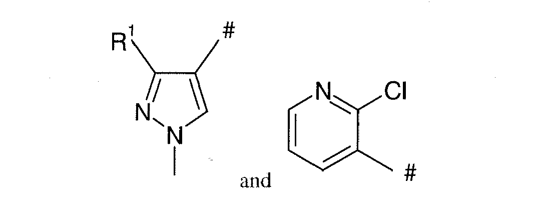 wherein "#" indicates the substituted position of the residue;
- R¹: is trifluoromethyl or difluoromethyl, more preferably difluoromethyl;
- R²: is cyano, nitro, fluorine, chlorine, bromine, methyl, ethyl, propyl, butyl, allyl, propargyl, methoxy, ethoxy, trifluoromethyl or phenyl, more preferably fluorine, chlorine, methyl or methoxy, most preferably fluorine or chlorine:
- R³: is methyl, ethyl, propyl, butyl, allyl or propargyl, more preferably methyl, ethyl or allyl, most preferably methyl;
- n: is 1 or 2, preferably 2.

The subsequent homogeneously catalyzed Suzuki biaryl cross-coupling is carried out according to scheme 2.

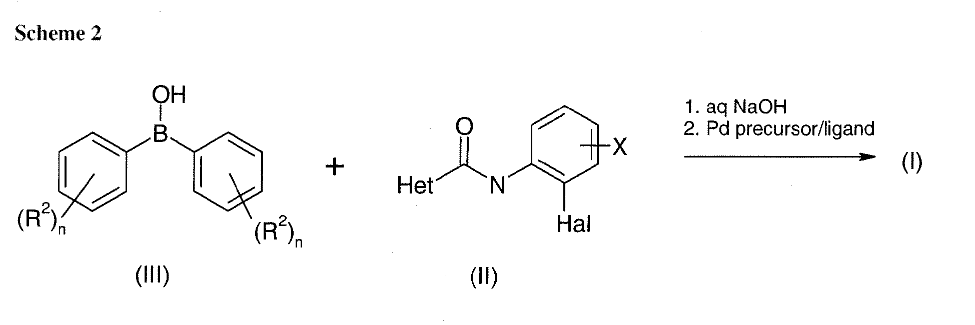

Preference is given to starting from diphenylborinic acids of the formula (III) in which R2 and n are as defined above.

Further preferred starting materials are diphenylborinic acids (III) in which n is 1 or 2, in particular 2. Particularly preferred are diphenylborinic acids (III) which are substituted in the 3- and 4-position or in 4-position only.

Very particular preference is given to di(2,3-diffuorophenyl)borinic acid, di(3,4-difluorophenyl)borinic acid, di(2,3-dich)orophenyl)borinic acid and in particular di(3,4-dichlorophenyl)borinic acid and (4-chlorophenyl)borinic acid as the starting compound (III).

Preference is given to starting from the following compounds (II):
N-(2-chloro-4-fluorophenyl)-3-(difluoromethyl)-1-methyl-1H-pyrazole-4-carboxamide, N-(2-bromo-4-fluorophenyl)-3-(difluoromethyl)-1-methyl-1H-pyrazole-4-carboxamide, N-(2-chloro-4-fluorophenyl)-3-(trifluoromethyl)-1-methyl-1H-pyrazole-4-carboxamide, N-(2-bromo-4-fluorophenyl)-3-(trifluoromethyl)-1-methyl-1H-pyrazole-4-carboxamide, N-(2-chloro-6-fluorophenyl)-3-(difluoromethyl)-1-methyl-1 H-pyrazole-4-carboxamide, N-(2-bromo-6-fluorophenyl)-3-(difluoromethyl)-1-methyl-1H-pyrazole-4-carboxamide, N-(2-chloro-6-fluorophenyl)-3-(trifluoromethyl)-1-methyl-1H-pyrazole-4-carboxamide, N-(2-bromo-6-fluorophenyl)-3-(trifluoromethyl)-1-methyl-1H-pyrazole-4-carboxamide,N-(2-chlorophenyl)-3-(difluoromethyl)-1-methyl-1H-pyrazole-4-carboxamide, N-(2-bromophenyl)-3-(difluoromethyl)-1-methyl-1H-pyrazole-4-carboxamide, N-(2-chlorophenyl)-3-(trifluoromethyl)-1-methyl-1H-pyrazole-4-carboxamide, N-(2-bromophenyl)-3-(trifluoromethyl)-1-methyl-1 H-pyrazole-4-carboxamide, 2-chloro-N-(2-chlorophenyl)pyridine-3-carboxamide, 2-chloro-N-(2-bromophenyl)pyridine-3-carboxamide particularly preferred are N-(2-chloro-4-fluorophenyl)-3-(difluoromethyl)-1-methyl-1H-pyrazole-4-carboxamide, N-(2-bromo-4-fluorophenyl)-3-(difluoromethyl)-1-methyl-1H-pyrazole-4-carboxamide, 2-chloro-N-(2-chlorophenyl)pyridine-3-carboxamide and 2-chloro-N-(2-bromophenyl)pyridine-3-carboxamide.

Compounds according to formula (II) can be prepared as described in WO 03/070705. Using, for example, 1-methyl-3-(trifluoromethyl)-1H-pyrazole-4-carbonyl chloride and 2-bromo-4-fluoroaniline as starting materials and a base as illustrated by the following equation:

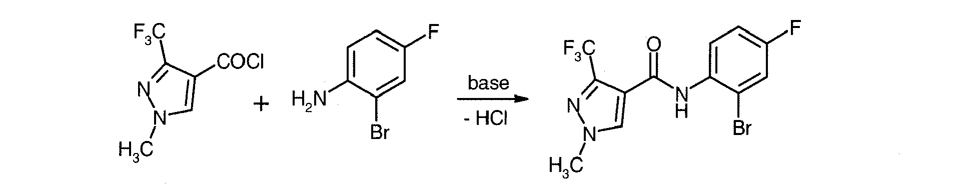

The compound (II) is used, based on the diphenylborinic acids (III) (diphenylborinic acid equivalents), normally in an equimolar amount, preferably with an up to 20 percent excess, in particular with an up to 50 percent excess. The bases used may be organic bases, for example tertiary amines. Preference is given to using, for example, triethylamine or dimethylcyclohexylamine. The bases used are preferably alkali metal hydroxides, alkaline earth metal hydroxides, alkali metal carbonates, alkaline earth metal carbonates, alkali metal hydrogencarbonates, alkali metal acetates, alkaline earth metal acetates, alkali metal alkoxides and alkaline earth metal alkoxides, in a mixture and in particular individually. Particularly preferred bases are alkali metal hydroxides, alkaline earth metal hydroxides, alkali metal carbonates, alkaline earth metal carbonates and alkali metal hydrogencarbonates. Especially preferred bases are alkali metal hydroxides, e.g. sodium hydroxide and potassium hydroxide, and also alkali metal carbonates and alkali metal hydrogencarbonates, e.g. lithium carbonate, sodium carbonate and potassium carbonate. The base is used in the process according to the invention preferably with a fraction of from 100 to 500 mol%, more preferably from 150 to 400 mol%, based on the amount of diphenylborinic acid (III). Suitable palladium catalysts are palladium-ligand complexes with palladium in the zero oxidation state, salts of palladium in the presence of complex ligands, or metallic palladium optionally applied to support, preferably in the presence of complex ligands. Suitable complex ligands are uncharged ligands such as triarylphosphines and trialkylphosphines, which may optionally be substituted in the aryl rings, such as triphenylphosphine (TPP), di-1-adamantyl-n-butylphosphine, tri-tert-butylphosphine (TtBP) or 2-(dicyclohexylphosphino)biphenyl.

Furthermore, the literature has also described further particularly reactive complex ligands from other structural classes, including 1 ,3-bis(2,6-diisopropylphenyl)-4,5-H2- imidazolium chloride (cf., for example, G. A. Grasa et al. Organometallics 2002, 21 , 2866) and tris(2,4-di-tert-butylphenyl) phosphite (cf. A. Zapf et al., Chem. Eur. J. 2000, 6, 1830).

The reactivity of the complex ligands can be enhanced by adding a quaternary ammonium salt such as tetra-n-butylammonium bromide (TBAB) (cf., for example, D. Zim et al., Tetrahedron Lett. 2000, 41, 8199). If required, the water solubility of the palladium complexes can be improved by various substituents such as sulfonic acid or sulfonate salt groups, carboxylic acid or carboxylate salt groups, phosphonic acid, phosphonium or phosphonate salt groups, peralkylammonium, hydroxyl and polyether groups. Among the palladiuin-ligand complexes with palladium in the 0 oxidation state, preference is given to using tetrakis(triphenylphosphine)palladium and additionally tetrakis[tri(o-tolyl)phosphine]palladium. In the salts of palladium which are used in the presence of complex ligands, the palladium is normally present in the two positive oxidation state. Preference is given to using palladium chloride, palladium acetate or bisacetonitrilepalladium chloride. Particular preference is given to using palladium chloride.

In general, from 6 to 60, preferably from 15 to 25, equivalents of the aforementioned complex ligands, in particular triphenylphosphine and tri-tert-butylphosphine, are combined with one equivalent of the palladium salt.

EP-A 0 888 261 describes the use of from 2 to 6 equivalents of triphenylphosphine per equivalent of the palladium catalyst. The use of high ligand excesses is generally viewed in the literature as disadvantageous, since this is expected to result in inactivation of the catalytically active complex (cf., for example, J. Hassan et al., Chem. Rev. 2002, 102, 1359). It was thus surprising that this high triphenylphosphine use in combination with the low catalyst use led to an increase in the overall yield of the process of the present invention and accordingly to an improvement in the economic viability. Metallic palladium is used preferably in pulverized form or on a support material, for example in the form of palladium on activated carbon, palladium on alumina, palladium on barium carbonate, palladium on barium sulfate, palladium on calcium carbonate, palladium aluminosilicates such as montmorillonite, palladium on SiO₂ and palladium on calcium carbonate, in each case with a palladium content of from 0.5 to 12% by weight. In addition to palladium and the support material, these catalyst may comprise further dopants, for example lead.

When metallic palladium optionally applied to support is used, particular preference is given to also using the aforementioned complex ligands, in particular to the use of palladium on activated carbon in the presence of triphenylphosphine as a complex ligand, where the phenyl groups in the triphenylphosphine are preferably substituted by a total of from one to three sulfonate groups. In the process according to the invention, the palladium catalyst is used with a low fraction of from 0.001 to 1.0 mol%, preferably from 0.005 to 0.5 mol% or from 0.01 to 0.5 mol% and in particular from 0.005 to 0.05 mol%, based on the amount of compound (II)

The low use of a palladium salt in combination with a high use of a complex ligand constitutes a significant cost advantage of this process over the prior art processes.

The process according to the invention may be carried out in a biphasic system composed of aqueous phase and solid phase, i.e. the catalyst. In that case, the aqueous phase may also comprise a water-soluble organic solvent in addition to water.

Organic solvents suitable for the process according to the invention are ethers such as dimethoxyethane, diethylene glycol dimethyl ether, tetrahydrofuran, dioxane and tert- butyl methyl ether, hydrocarbons such as n-hexane, n-heptane, cyclohexane, benzene, toluene and xylene, alcohols such as methanol, ethanol, 1-propanol, 2-propanol, ethylene glycol, 1-butanol, 2-butanol and tert.-butanol, ketones such as acetone, ethyl methyl ketone and isobutyl methyl ketone, amides such as dimethylformamide, dimethylacetamide and N-methylpyrrolidone, in each case individually or in a mixture.

Preferred solvents are ethers such as dimethoxyethane, tetrahydrofuran and dioxane, hydrocarbons such as cyclohexane, toluene and xylene, alcohols such as ethanol, 1- propanol, 2-propanol, 1-butanol and tert.-butanol, in each case individually or in a mixture. In a particularly preferred variant of the process according to the invention, water, one or more water-insoluble and one or more water-soluble solvents are used, for example mixtures of water and dioxane, or water and tetrahydrofuran, or water, dioxane and ethanol, or water, tetrahydrofuran and methanol, or water, toluene and tetrahydrofuran, preferably water and tetrahydrofuran, or water, tetrahydrofuran and methanol.

The total amount of solvent is normally from 3000 to 500 g and preferably from 2000 to 700 g, per mole of the compound (II).

Appropriately, the process is carried out by adding the compound (II), the diphenyl- borinic acids (III), the base and the catalytic amount of the palladium catalyst to a mixture of water and one or more inert organic solvents, and stirring at a temperature of from 50°C to 120°C, preferably from 70°C to 110°C, more preferably from 90°C to 100°C, for a period of from 1 to 50 hours, preferably from 2 to 24 hours.

Depending on the solvent and temperature used, a pressure of from I bar to 6 bar, preferably from 1 bar to 4 bar, is established. Preference is given to carrying out the reaction in water and tetrahydrofuran. The reaction may be carried out in customary apparatus suitable for such processes. On completion of reaction, palladium catalyst obtained as a solid is removed, for example by filtration, and the crude product is freed from the solvent or the solvents. In the case of products which are not fully water-soluble, water-soluble palladium catalysts or complex ligands are removed fully from the crude product in the separation of the water phase. Subsequently, further purification may be effected by methods which are known to those skilled in the art and are appropriate to the particular product, for example by recrystallization, distillation, sublimation, zone melting, melt crystallization or chromatography.

By the process according to the invention, it is possible to prepare, for example:
2-chloro-N-(4'-chlorobiphenyl-2-yl)pyridine-3-carboxamide, N-(3',4'-dichlorobipheny(-2-yl)-3-(difluoromethyl)-1-methyl-1H-pyrazole-4-carboxamide, N-(3',4'-dichloro-5-fluorobiphenyl-2-yl)-3-(difluoromethyl)-1-methyl-1H-pyrazole-4-carboxamide, 3-(trifluoromethyl)-1-methyl-1H-pyrazole-4-carboxamide, N-(3',4'-dichloro-5-fluorobiphenyl-2-yl)-3-(trifluoromethyl)-1-methyl-1H-pyrazole-4-carboxamide.

The process according to the invention affords the compounds I in very high up to quantitative yields at very good purity. The substituted biphenyls obtainable by the process according to the invention are suitable as precursors for fungicidal crop protection active ingredients (cf. WO 03/070705).

## Claims

1. A process for preparing substituted biphenylanilides of the formula I 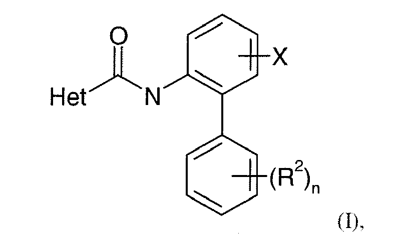
Het is a heterocyclyl-residue selected from 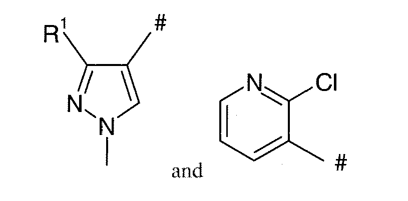 wherein "#" indicates the substituted position of the residue:
X is hydrogen fluorine, or chlorine;
R¹ is C₁-C₆-haloalkyl.,
R² is cyano, nitro, halogen, C₁-C₆-alkyl, C₁-C₆-alkenyl, C₁-C₆-alkynyl, C₁-C₆-alkoxy, C₁-C₆-haloalkyl, (C₁-C₆-alkyl)carbonyl or phenyl;
R³ is C₁-C₄-alkyl. C₁-C₄-aJkenyl or C₁-C₄-alkynyl;
n is 1, 2 or 3, where in case that n is 2 or 3, the R² radicals may also be different,
which comprises reacting a compound of formula II 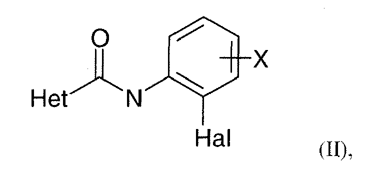 in which Hal is halogen and X and Het are as defined above, in the presence of a base and of a palladium catalyst selected from the group of:
a) palladium-triarylphosphine or -trialkylphosphine complex with palladium in the zero oxidation state.
b) salt of palladium in the presence of triarylphospine or trialkylphosphine as a complex ligand or
c) metallic palladium, optionally applied to support, in the presence of triarylphosphine or trialkylphosphine,
in a solvent, with a diphenylborinic acid according to formula (III) 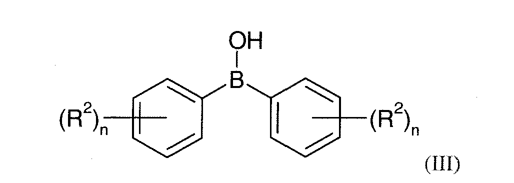 in which R² and n are as defined above, where the triarylphosphines or trialkylphosphines used may be substituted.

2. The process according to claim 1, wherein the compound (II) used is selected from the group consisting of N-(2-chloro-4-fluorophenyl)-3-(difluoromethyl)-1-methyl-1H-pyrazole-4-carboxamide, N-(2-bromo-4-fluorophenyl)-3-(difluoromethyl)-1-methyl-1H-pyrazole-4-carboxamide, 2-chloro-N-(2-chlorophenyl)pyridine-3-carboxamide and 2-chloro-N-(2-bromophenyl)pyridine-3-carboxamide.

3. The process according to claim 1 or 2, wherein the starting compound (III) is a diphenylborinic acid which is substituted in the 3- and 4-position.

4. The process according to claims 1 or 2, wherein a diphenylborinic acid (III) is used which bears fluorine or chlorine in the 3- and 4-positions.

5. The process according to claims I or 2, wherein the starting compound (III) is di(3,4-dichlorophenyl)borinic acid.

6. The process according to claims 1 to 5, wherein the palladium catalyst a) according to claim 1 used is tetrakis(triphenylphosphine)palladium or tetralcis(tri-tert.-butylphosphine)palladium.

7. The process according to claims I to 5, wherein a palladium catalyst b) according to claim 1 is used.

8. The process according to claims 1 to 5, wherein the palladium catalyst c) according to claim 1 used is metallic palladium on activated carbon in the presence of triphenylphosphine whose phenyl groups are substituted by a total of from 1 to 3 sulfonate groups.

9. The process as claimed in claim 7, wherein the salt of the palladium catalyst b) used is palladium chloride, palladium acetate or bisacetonitrilepalladium chloride.

10. The process according to claim 7, wherein a palladium catalyst b) is used for which from 6 to 60 equivalents of triphenylphosphine are used per equivalent of the palladium salt.

11. The process according to claim 1, wherein from 0.001 to 1.0 mol% of the palladium catalyst is used, based on the amount of compound (II).

12. The process according to claim 1, wherein the reaction is carried out at a temperature of from 50 to 120°C.

13. The process according to claim 1 , wherein the reaction is carried out in a mixture of water and an organic solvent.

14. The process according to claim 13, wherein the organic solvent used is an ether.

15. The process according to claim 1, wherein the reactions are carried out at a pressure of from 1 to 6 bar.

16. The process according to claim 1, wherein compound (II) is N-(2-chloro-4-fluorophenyl)-3-(difluoromethyl)-1-methyl-1H-pyrazole-4-carboxamide and compound (III) is di(3,4-dichlorophenyl)borinic acid.

17. The process according to claim 1, wherein compound (II) is N-(2-chlorophenyl)-3-(difluoromethyl)-1-methyl-1H-pyrazole-4-carboxamide and compound (III) is di(3,4-dichlorophenyl)borinic acid.

18. The process according to claim 1, wherein compound (II) is 2-chloro-N-(2-bromophenyl)pyridine-3-carboxamide and compound (III) is (4-chlorophenyl)borinic acid.
